# EUROPEAN PATENT APPLICATION

(11) **EP 1 944 012 A1**
(43) Date of publication of application: **16.07.2008**
(21) Application number: 06810853.9
(22) Date of filing: 29.09.2006
(51) Int. Cl.: A61K 8/49, A61K 8/89, A61Q 17/04

(54) **SUNSCREEN COSMETIC COMPOSITION**

(30) Priority: 05.10.2005 JP 2005292493; 05.10.2005 JP 2005292494
(71) Applicant: Shiseido Company, Limited, Chuo-ku Tokyo 104-8010 (JP)
(72) Inventor: ABE, Koji, Yokohama-shi Kanagawa 224-8558 (JP); OGUCHI, Nozomi, Yokohama-shi Kanagawa 224-8558 (JP); YAJIMA, Isao, Yokohama-shi Kanagawa 224-8558 (JP); ARAKI, Hidefumi, Yokohama-shi Kanagawa 224-8558 (JP); KAKOKI, Hiroyuki, Yokohama-shi Kanagawa 224-8558 (JP)
(74) Representative: Merkle, Gebhard
(86) International application number: PCT/JP2006/319447
(87) International publication number: WO 2007/040171

(57) **Abstract**

The present invention is a sunscreen cosmetic comprising 2,4-bis [[4-(2-ethylhexyloxy)-2-hydroxy] phenyl]-6-(4-methoxyphenyl)-1,3,5-triazine and a specific benzotriazole derivative.

Also, the present invention is a sunscreen cosmetic comprising a specific benzotriazole derivative and a specific alkylaryl 1,3-propanedione silicone derivative.

An object of the present invention is to provide a sunscreen cosmetic that exhibits superior UV-A absorption capacity and excellent stability and also sufficiently dissolves 2,4-bis [[4-(2-ethylhexyloxy)-2-hydroxy] phenyl]-6-(4-methoxyphenyl)-1,3,5-triazine, which has low solubility and crystallizes easily.

Another object is to provide a sunscreen cosmetic that can synergistically improve the ultraviolet absorption capacity of conventional ultraviolet absorbents.

## Description

### TECHNICAL FIELD

The present invention relates to a sunscreen cosmetic. More specifically, it relates to a sunscreen cosmetic that has superior capacity to dissolve specific solid powder ultraviolet absorbents and also has particularly superior capacity to absorb the UV-A region. (First invention)
It also relates to a sunscreen cosmetic that synergistically augments absorption capacities in the UV-A region and the UV-B region. (Second invention)

### BACKGROUND ART

Important ultraviolet wavelength regions absorbed by sunscreen cosmetics are the UV-A region (320-400 nm) and UV-B region (290-320 nm). It was believed that the ultraviolet light in the UV-A region (320-400 nm) darkened the skin but it would not cause sunburn and accelerate aging of the skin as the ultraviolet light in the UV-B region (290-320 nm) would. However, in recent years, it has been made clear that, whereas the ultraviolet light in the UV-B region only reaches the surface part of the skin, the ultraviolet light in the UV-A region reaches the deeper part of the skin and induces not only skin aging but also skin cancer.

Ultraviolet absorbents for cosmetics that have been used up to the present are structurally categorized into (1) benzoic acid derivatives, (2) cinnamic acid derivatives, (3) benzophenone derivatives, (4) dibenzoylmethane derivatives, and (5) salicylic acid derivatives. In recent years, ultraviolet absorbents of (2) and (4) are frequently used.

However, the ultraviolet absorbents listed above each have problems from a practical point of view. For example, 2-ethylhexyl-p-dimethylaminobenzoate, an example of (1) benzoic acid derivatives, is a transparent liquid and has the advantage of being easy to handle; however, it and its derivatives are questionable in terms of safety and therefore are not used in recent years. Also, its peak absorption wavelength is near 290 nm and it absorbs only the ultraviolet light in the UV-B region.

Among (2) cinnamic acid derivatives, 2-ethylhexyl-p-methoxycinnnamate is the most frequently used ultraviolet absorbent in sun care cosmetics currently available commercially. Its maximum absorption wavelength is near 310 nm and its absorption region does not reach the UV-A region. Also, sun light degenerates it and therefore it has problems with staining and also with instability of the ultraviolet protection effect.

As for (3) benzophenone derivatives, 2-hydroxy-4-methoxybenzophenone, for example, absorbs both the UV-A and UV-B regions and exhibits relatively good solubility in endermic liniment base agents; however, its maximum absorption wavelength is rather close to the UV-B region and the absorbance is not very high. Also, in recent years, its basic structural skeleton (benzophenone) has been implicated as an environmental hormone and its use has been avoided.

Among (4) dibenzoylmethane derivatives, 4-tert-buthoxy-4-methoxybenzoylmethane is frequently used for endermic liniments. Its maximum absorbance is at around 360 nm and the absorbance level is high, and therefore it is a superior ultraviolet absorbent in the UV-A region. However, it has a problem in photostability, and it exhibits poor compatibility with oil components in endermic liniments and therefore only a small amount can be added.

Among (5) salicylic acid derivatives, octyl salicylate is used. It has the maximum absorption wavelength in the UV-B region, and it is in an oil form and exhibits superior compatibility with paraffin oil and such; however, since its absorbance is low, it is not put to practical use much.

Therefore, 2-ethylhexyl-p-methoxycinnamate from (2) is often used in the UV-B region and 4-tert-buthoxy-4-methoxybenzoylmethane from (4) is often used in the UV-A region. In recent years in particular, there is an increasing demand for ultraviolet absorption in the UV-A region.

In contrast, an endermic liniment that contains, for a ultraviolet absorbent, a benzotriazole derivative represented by the general formula (1-I) or (2-I), exhibits superior compatibility with oil components of various endermic liniment base agents, superior ultraviolet absorption in the UV-A region, and superior photostability of the ultraviolet absorbent itself, and also is capable of a lasting ultraviolet absorption effect has been developed (Patent Document 1).

On the other hand, 2,4-bis [[4-(2-ethylhexyloxy)-2-hydroxy] phenyl]-6-(4-methoxyphenyl)-1,3,5-triazine is a prior art ultraviolet absorbent having a superior UV-A absorption capacity and it is commercially available with the product name Tinosorb (registered trademark) S from Ciba Specialty Chemicals Holding Inc.

However, this ultraviolet absorbent is powder and its solubility in oil components is low: and even when dissolved it tends to recrystallize. Therefore, it cannot manifest sufficient ultraviolet absorption capability when blended into a cosmetic. Also, crystals would precipitate over time, which makes it very difficult to manufacture stable cosmetics.

To avoid this problem, solubilizing oil components that can be added to cosmetics have been investigated but the results are not sufficient. For example, in Patent Document 2, ethylhexyl 2-cyano-3,3-diphenylacrylate (octocrylene) is used to solubilize it; however, this is not necessarily a sufficient solubilizing oil component. Also, this oil component itself does not necessarily contribute to the UV-A absorption.
On the other hand, an endermic liniment that contains the alkylaryl-1,3-propanedione silicone derivative represented by the general formula (2-II) is disclosed in Patent Document 3 and Patent Document 4.

Patent Citation 1: Japanese Patent Laid-Open 2005-206473 bulletin
Patent Citation 2: Japanese Patent Laid-Open 2000-309520 bulletin
Patent Citation 3: Japanese Patent Laid-Open H5-194543 bulletin
Patent Citation 4: Japanese Patent Laid-Open H11-269050 bulletin

### DISCLOSURE OF INVENTION

### TECHNICAL PROBLEM

### [First invention: Invention of claims 1-2]

Based on the point of view described above, the inventors conducted earnest research and discovered that the benzotriazole derivative of the general formula (1-I), which exhibits superior ultraviolet absorption in the UV-A region, could be used as an excellent solubilizing oil component for 2,4-bis [[4-(2-ethylhexyloxy)-2-hydroxy] phenyl]-6-(4-methoxyphenyl)-1, 3, 5-triazine and accordingly prepared a sunscreen cosmetic that exhibits superior ultraviolet absorption capacity in the UV-A region and superior stability, thus completing the present invention.

### [Second invention: Invention of claims 3-5]

As described above, it is very important for a sunscreen cosmetic to maintain high ultraviolet absorption capacity in a broad region covering the UV-A and UV-B. To achieve this, ultraviolet absorbents exhibiting absorption capacity in the UV-A region and in the UV-B region are combined. However, simply using two ultraviolet absorbents in combination does not produce an effect exceeding the absorption capacity of each absorbent. Also, for a sunscreen cosmetic, a combination that produces the maximum effect with small blend ratios of the ultraviolet absorbents is desirable.

Based on the point of view described above, the inventors discovered that combined use of a benzotriazole derivative of a specific structure and a specific alkylaryl-1,3-propanedione silicone derivative synergistically improved ultraviolet absorption capacity in the UV-A and UV-B regions and accordingly solved the problems mentioned above, thus completing the present invention.

### TECHNICAL SOLUTION

### [First invention]

That is, the present invention provides a sunscreen cosmetic comprising 2,4-bis [[4-(2-ethylhexyloxy)-2-hydroxy] phenyl]-6-(4-methoxyphenyl)-1,3,5-triazine and a benzotriazole derivative represented by the following general formula (1-I) . 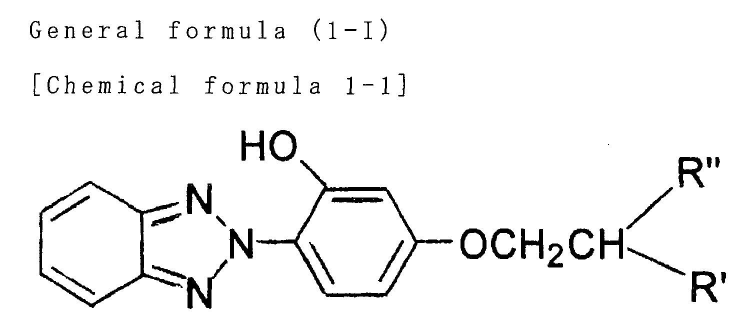 (In this formula, R' = a straight chain alkyl group of C1-C6 and R" = a straight chain alkyl group of C1-C3.)

Also, the present invention provides the aforementioned sunscreen cosmetic wherein said benzotriazole derivative is one, two or more chosen from a group consisting of 2-[4-(2-ethylhexyloxy)-2-hydroxyphenyl]-2H-benzotriazole and 2-(2-hydroxy-4-isobuthoxyphenyl)-2H-benzotriazole.

### [Second invention]

That is, the present invention provides a sunscreen cosmetic comprising a benzotriazole derivative represented by the following general formula (2-I) and an alkylaryl 1,3-propanedione silicone derivative represented by the following general formula (2-II). 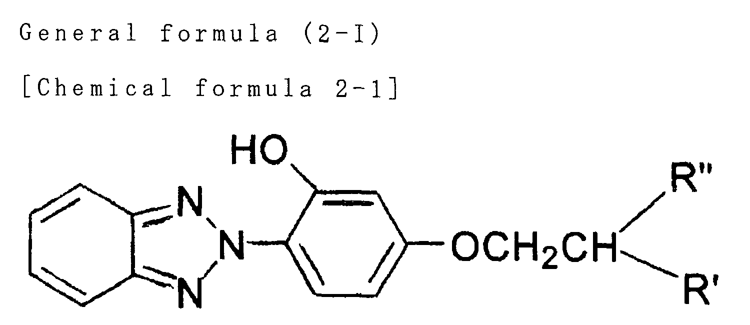 (In this formula, R' = a straight chain alkyl group of C1-C6 and R" = a straight chain alkyl group of C1-C3.) 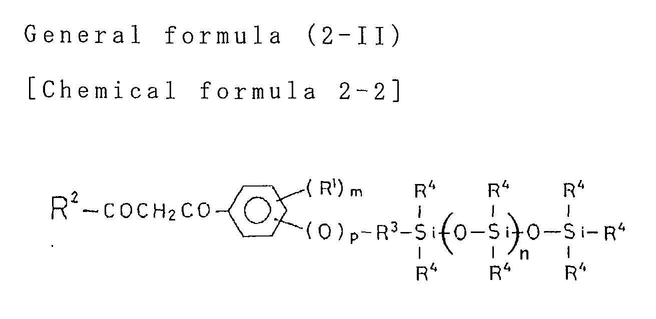 (R¹ denotes a hydroxyl group, an alkyl group having 1-8 carbons or an alkoxy group having 1-8 carbons, R² denotes an alkyl group having 1-7 carbons, R³ denotes a divalent alkylene group or oxyalkylene group having 2-10 carbons, R⁴s can be identical or different from each other and each denote an alkyl group having 1-4 carbons, phenyl group, or trimethylsiloxy group, m and n denote integers 0-3, and p denotes 0 or 1.)

Also, the present invention provides the aforementioned sunscreen cosmetic wherein said benzotriazole derivative is one, two or more chosen from a group consisting of 2-[4-(2-ethylhexyloxy)-2-hydroxyphenyl]-2H-benzotriazole and 2-(2-hydroxy-4-isobuthoxyphenyl)-2H-benzotriazole.

Furthermore, the present invention provides the aforementioned sunscreen cosmetic wherein said alkylaryl 1,3-propanedione silicone derivative is a chemical compound represented by the following formula (2-III) or (2-IV).

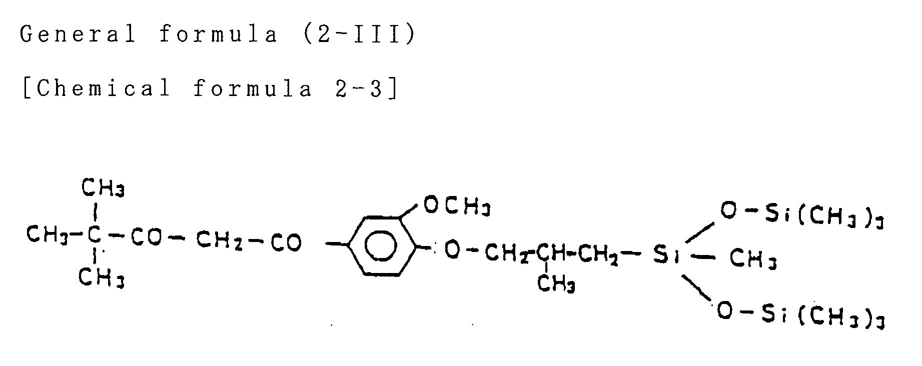

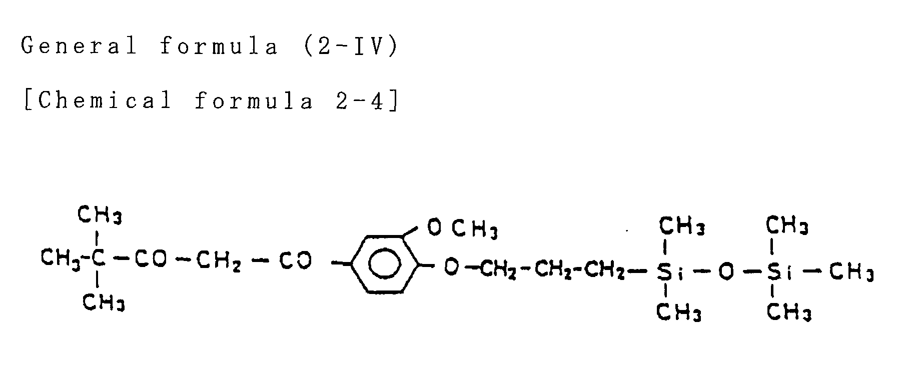

### ADVANTAGEOUS EFFECTS

### [First invention]

The sunscreen cosmetic of the present invention contains a benzotriazole derivative represented by the general formula (1-I) as the oil component to dissolve 2,4-bis [[4-(2-ethylhexyloxy)-2-hydroxy] phenyl]-6-(4-methoxyphenyl)-1,3,5-triazine, which is an excellent ultraviolet absorbent in the UV-A region, and thus this cosmetic can sufficiently dissolve said ultraviolet absorbent in the powder form.
As a result, said ultraviolet absorbent can manifest sufficient UV-A ultraviolet absorption capacity and said ultraviolet absorbent does not precipitate as crystals, making it possible to provide a sunscreen cosmetic exhibiting superior product stability.
Also, the benzotriazole derivative of general formula (1-I), i. e. the solubilizing oil component, has its own superior UV-A ultraviolet absorption capacity; therefore together they can manifest a high ultraviolet absorption capacity. Because of this, there is also an advantage in that the blend ratio of the ultraviolet absorbents can be smaller compared with other sunscreen cosmetics in order to manifest the same degree of ultraviolet absorption capacity.

### [Second invention]

The sunscreen cosmetic of the present invention can synergistically improve the ultraviolet absorption capacity of conventional ultraviolet absorbents. As a result, a sunscreen cosmetic that exhibits high ultraviolet absorption capacity over a broad region from UV-A to UV-B can be provided. That is, the sunscreen cosmetic of the present invention utilizes a combination of ultraviolet absorbents that maximizes the absorption effect using small blend ratios. Also, the sunscreen cosmetic of the present invention has the effect of improving the sensation during use (sticky sensation) of a sunscreen cosmetic containing a benzotriazole derivative represented by general formula (2-1).

### BRIEF DESCRIPTION OF DRAWINGS

### [First invention]

[FIG. 1] FIG. 1 is a graph showing solubility of 2,4-bis [[4-(2-ethylhexyloxy)-2-hydroxy] phenyl]-6-(4-methoxyphenyl)-1,3,5-triazine in various oil components.

### [Second invention]

[FIG. 2] FIG. 2 shows ultraviolet absorption spectra (absorbance) showing absorption characteristics in the UV-A and UV-B regions.
[FIG. 3] FIG. 3 shows ultraviolet absorption spectra (absorbance) showing absorption characteristics in the UV-A and UV-B regions.
[FIG. 4] FIG. 4 shows ultraviolet absorption spectra (absorbance) showing absorption characteristics in the UV-A and UV-B regions.
[FIG. 5] FIG. 5 shows ultraviolet absorption spectra (absorbance) showing absorption characteristics in the UV-A and UV-B regions.
[FIG. 6] FIG. 6 shows ultraviolet absorption spectra (absorbance) showing absorption characteristics in the UV-A and UV-B regions.

### BEST MODE FOR CARRYING OUT THE INVENTION

The present invention is described in detail below.

### [First invention]

2,4-bis [[4-(2-ethylhexyloxy)-2-hydroxy] phenyl]-6-(4-methoxyphenyl)-1,3,5-triazine is commercially available under the product name Tinosorb (registered trademark) S from Ciba Specialty Chemicals Holding Inc. It is a UV-A ultraviolet absorbent in a powder form.

### "Benzotriazole derivative represented by general formula (1-I)"

The benzotriazole derivative of general formula (1-I) is a prior art chemical compound, which is synthesized in the following manner. A common method is to use sodium nitrite and such to turn o-nitroaniline into a diazonium salt and then couple this with phenol to synthesize a monoazo compound, and reduce it to obtain benzotriazole.

### (Method A)

### First process

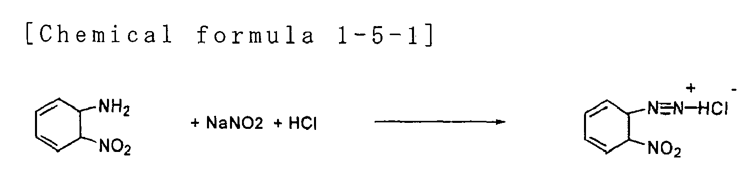

### Second process

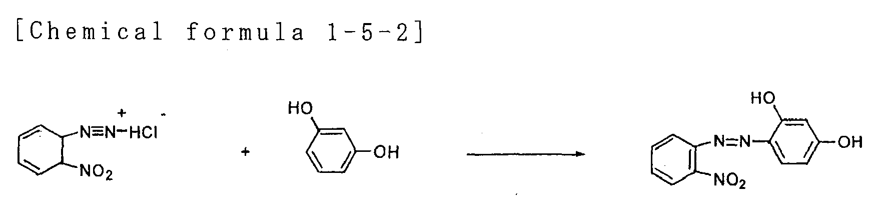

### Third process

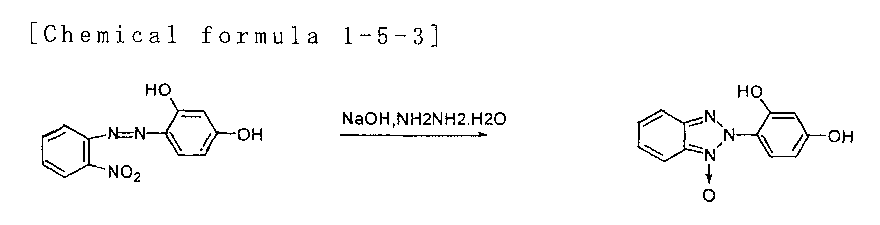

### Fourth process

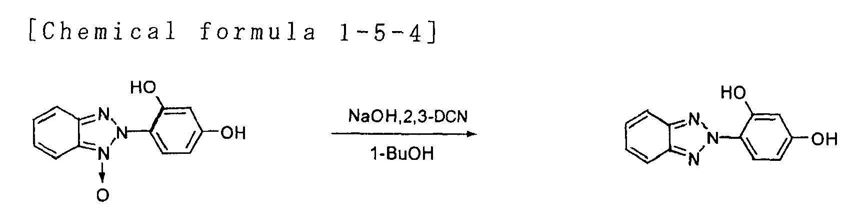

(In this formula, 2,3-DCN denotes 2,3-dichloro-1,4-naphthoquinone.)

### Fifth process

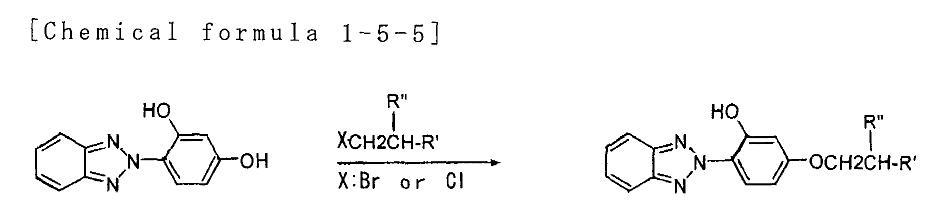

(In this formula, R = H or CH3, R' = a straight chain alkyl group of C1-C6 and R" = a straight chain alkyl group of C1-C3.)

### (Method B)

### First process

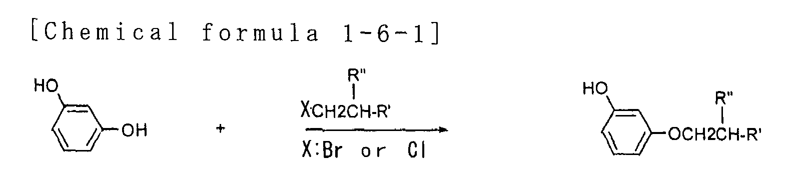

### Second process

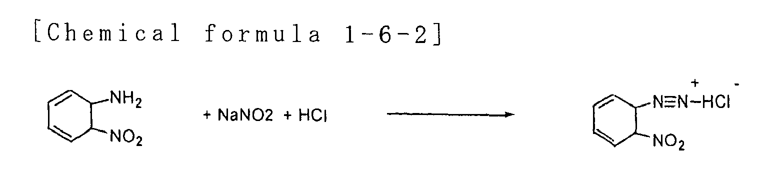

### Third process

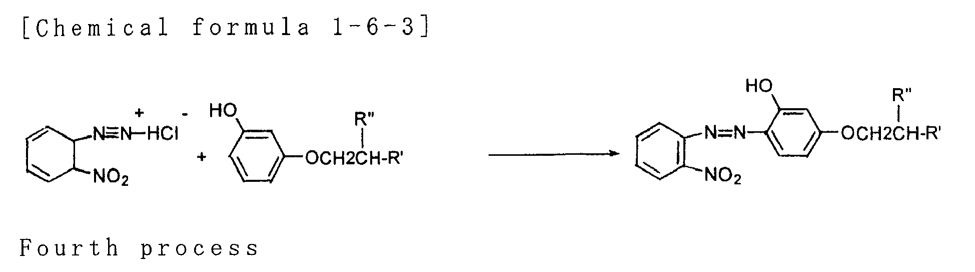

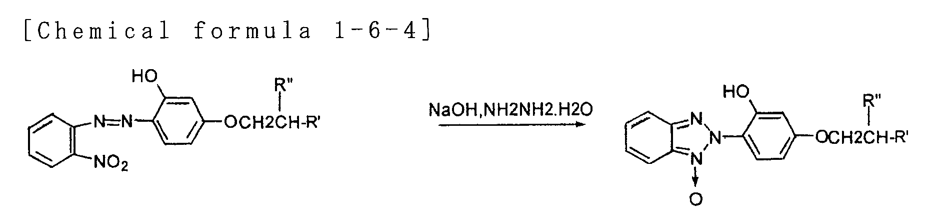

### Fifth process

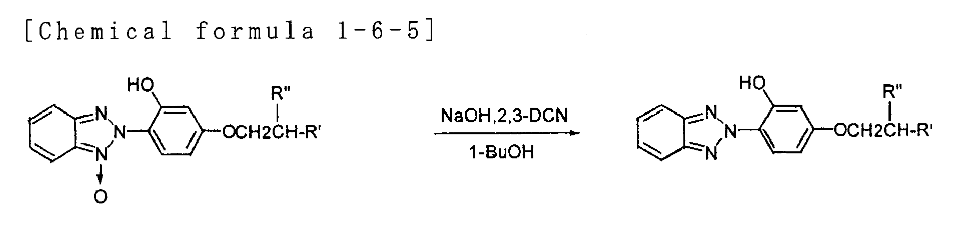

(In this formula, R' = a straight chain alkyl group of C1-C6 and R" = a straight chain alkyl group of C1-C3. 2,3-DCN denotes 2, 3-dichloro-1, 4-naphthoquinone.)

Refluxing a corresponding benzotriazole and alkylated halogen in a mixed solvent of methylisobutylketone and dimethylformamide produces the compound of general formula (1-I) with a particularly high yield.
Specifically, 6-(2H-bonzotriazole-2-yl) resorcinol is put into a four-neck flask equipped with a thermometer and refluxing cooler, to which methylisobutylketone and dimethylformamide are added and the mixture is stirred. To this, sodium carbonate and 2-ethylhexyl bromide are added and the temperature is raised to the refluxing temperature while stirring. After the mixture is stirred while the refluxing temperature is maintained for a prescribed amount of time, methylisobutylketone is recovered under normal pressure; the remaining oil is then rinsed with water to remove excess sodium carbonate and inorganic byproducts to obtain liquid 2-[4-(2-ethylhexyloxy)-2-hydroxyphenyl]-2H-benzotriazole at a high yield.

The blend ratio of 2,4-bis [[4-(2-ethylhexyloxy)-2-hydroxy] phenyl]-6-(4-methoxyphenyl)-1,3,5-triazine is chosen as appropriate for the target product; it is preferably 0.5-5 wt%, more preferably 1-3 wt%, of the total amount of the sunscreen cosmetic.

The blend ratio of the benzotriazole derivative represented by general formula (1-I) is chosen as appropriate for the blend ratio of 2,4-bis [[4-(2-ethylhexyloxy)-2-hydroxy] phenyl]-6-(4-methoxyphenyl)-1,3,5-triazine to be dissolved. Usually it is preferably 2-10 wt%, more preferably 3-7 wt%, of the total amount of the sunscreen cosmetic.

### [Second invention]

### "Benzotriazole derivative represented by general formula (2-I)"

The benzotriazole derivative of general formula (2-I) is a prior art chemical compound. It is the same as the chemical compound of general formula (1-I), which is used in the aforementioned

### [First invention].

### "Alkylaryl-1,3-propanedione silicone derivative represented by general formula (2-II)"

The alkylaryl-1,3-propanedione silicone derivative of general formula (2-II) is a prior art chemical compound (see Japanese Patent Publication No. 3229383 bulletin).
Examples of R¹ include a hydroxyl group, alkyl groups such as methyl group, ethyl group, propyl group, isopropyl group, butyl group, isobutyl group, sec-butyl group, t-butyl group, n-amyl group, isoamyl group, n-hexyl group, 2-ethylbutyl group, n-octyl group, and 2-ethylhexyl group, and alkoxy groups such as a methoxy group, ethoxy group, propoxy group, isopropoxy group, butoxy group, isobutoxy group, s-butoxy group, t-butoxy group, n-amyloxy group, isoamyloxy group, n-hexyloxy group, 2-ethylbutoxy group, n-pentyloxy group, n-octyloxy group, and 2-ethylhexyloxy group.
R² can be any alkyl group having 1-7 carbon atoms; the carbon atom bonded to the carboxyl group is preferably secondary or tertiary. Particularly preferable examples of R² include an isopropyl group, tert-butyl group, ethylpropyl group, and ethylpentyl group.
R³ can be any alkyl group having 2-10 carbon atoms. Examples of R³ include -CH₂CH₂-,-CH₂CH₂CH₂-, -CH₂CH(CH₃)CH₂-, -CH(CH₃)CH₂CH₂-,-CH₂CH₂CH(CH₃)-, -CH₂CH₂CH₂CH₂-, -CH₂CH₂OCH₂CH₂-, hexylene, cyclohexylene, and decylene.
Particularly preferable are alkylene groups having 2-4 carbon atoms. Examples of R⁴ include a methyl group, ethyl group, propyl group, isopropyl group, butyl group, isobutyl group, t-butyl group, phenyl group, and trimethylsiloxy group; from the point of view of the ease of procuring raw materials, it is preferable to use mainly methyl groups with some phenyl groups, or trimethylsiloxy groups.

Also, m denotes a number of substituents and is an integer 0-3. n denotes the number of repeating units of siloxane and is an integer 0-3. p is 0 or 1.
R^{4'} s can be identical to or different from each other; examples include a methyl group, ethyl group, propyl group, isopropyl group, butyl group, isobutyl group, t-butyl group, phenyl group, and trimethylsiloxy group; from the point of view of the ease of procuring raw materials, it is preferable to use mainly methyl groups with some phenyl groups, or trimethylsiloxy groups.

The alkylaryl-1,3-propanedione silicone derivative used in the present invention can be liquid or a resin-like solid at room temperature, depending on the molecular weight; in either case it can be used as an UV-A absorbent. The alkylaryl-1,3-propanedione silicone derivative of the present invention can be synthesized by the following two-step reaction including the first step which is a Claisen condensation reaction and the second step which is a hydrosilylation reaction.

### First step

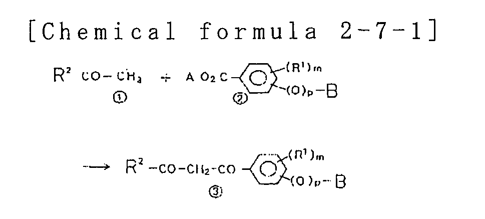

or

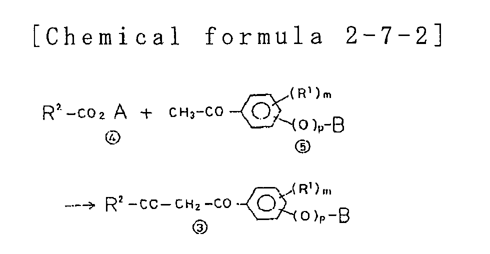

### Second step

### {Compound of the aforementioned circled number 3} + {Siloxane having 2-5 repeating units and containing one Si-H group in a molecule} ---> General formula (2-II)

In the aforementioned reaction formula, R', R², m, n, and p are as defined in the aforementioned chemical formula 2, A denotes an alkyl group having 1-5 carbons, B denotes an alkenyl or oxyalkenyl group having 2-10 carbons. The Claisen condensation reaction, i.e. the first step, is described in Hauer, R., "Organic Reactions", John Wiley and Sons Inc., New York, vol. 8, p59, 1954. In the present invention this description was followed.

That is, methyl alkyl ketone (circled number 1) or substituted acetophenone (circled number 5) and fatty acid or substituted alkylphenyl benzoate (circled number 4) or the compound of circled number 2 can be reacted at a temperature between room temperature and the boiling temperature in the presence of a base, such as alkali alcoholates, hydroxides or amide compound and sodium hydride, in a solvent such as toluene, isopropyl ether, tetrahydrofuran, 1,2-dimethoxyethane, dimethylsulfoxide or dimethylformamide, to obtain an alkyl-aryl-1,3-propanedione derivative (circled number 3). The second step reaction is a hydrosilylation reaction. This reaction is known to be accelerated by a platinum group metal, a chemical compound of a platinum group metal, or a complex compound of such a metal (see Japanese Patent Laid-Open No. S60-108431 bulletin, Japanese Patent Laid-Open No. S60-216032 bulletin, and Japanese Patent Laid-Open No. H1-50711 bulletin).
The reaction between the alkylaryl-1,3-propanedione derivative (circled number 3) and siloxane having 2-5 repeating units and containing one Si-H group is also accelerated by usual catalysts such as platinum supported on carbon, chloroplatinic acid, a platinum complex of acetylacetone, a platinum complex of an unsaturated compound, a platinum complex of unsaturated siloxanes, a rhodium compound, and a complex compound of a platinum compound.

The blend ratio of the benzotriazole derivative represented by general formula (2-I) is chosen as appropriate for the target product; it is preferably 0.5-7 wt%, more preferably 1-5 wt%, of the total amount of the sunscreen cosmetic.
The blend ratio of the alkylaryl 1,3-propanedione silicone derivative represented by general formula (2-II) is chosen as appropriate for the target product; it is preferably 0.5-10 wt%, more preferably 1-7 wt%, of the total amount of the sunscreen cosmetic.

### [First and second inventions]

In addition to the aforementioned essential ingredients, other ingredients used in endermic liniments such as cosmetics or medical drugs can be blended in as necessary in the sunscreen cosmetic of the present invention; examples of such ingredients include whitening agents, humectants, antioxidants, oil-based ingredients, other ultraviolet absorbents, surfactants, thickeners, alcohols, powder ingredients, coloring agents, water-based ingredients, water, and various skin nutrients, and the sunscreen cosmetic can be prepared with a conventional method. The following are some examples of the ingredients.

Oil components such as avocado oil, macadamia nut oil, corn oil, olive oil, rapeseed oil, evening primrose oil, castor oil, sunflower oil, tea seed oil, rice bran oil, jojoba oil, cacao oil, coconut oil, squalene, beef tallow, Japanese core wax, beeswax, candelilla wax, carnauba wax, whale wax, lanolin, liquid paraffin, polyoxyethylene (8 mole) oleyl alcohol ether, glyceryl monooleate, cyclomethicone, dimethylpolysiloxane, and diphenylpolysiloxane.
Higher alcohols such as caprylic alcohol, lauryl alcohol, myristyl alcohol, cetyl alcohol, cholesterol, and phytosterol.
Higher fatty acids such as caprylic acid, lauric acid, myristic acid, palmitic acid, stearic acid, behenic acid, lanolin fatty acid, linoleic acid, and linolenic acid.
Humectants such as polyethylene glycol, glycerin, sorbitol, xylitol, maltitol, mucopolysaccharide, hyaluronic acid, chondroitin sulfate, and chitosan.
Thickeners such as methyl cellulose, ethyl cellulose, Arabic gum, and polyvinyl alcohol.
Organic solvents such as ethanol and 1,3-butylene glycol.
Antioxidants such as butylhydroxytoluene, tocopherol, and phytic acid.
Antibacterial preservatives such as benzoic acid, salicylic acid, sorbic acid, paraoxybenzoic esters (ethylparaben and butylparaben, for example), and hexachlorophene.
Amino acids such as glycine, alanine, valine, leucine, serine, threonine, phenyalanine, tyrosine, aspartic acid, asparagine, glutamine, taurine, arginine, and histidine, as well as hydrochlorides thereof.
Organic acids such as acyl sarcosinic acid (sodium lauroyl sarcosinate, for example), glutathione, citric acid, malic acid, tartaric acid, and lactic acid.
Vitamins such as vitamin A and its derivatives, vitamin B's including vitamin B6 hydrochloride, vitamin B6 tripalmitate, vitamin B6 dioctanoate, vitamin B2 and its derivatives, vitamin B12, and vitamin B15 and its derivatives, vitamin C's including ascorbic acid, ascorbic malic esters (salts), and ascorbic dipalmitate, vitamin E' s including α-tocopherol, β-tocopherol, γ-tocopherol, vitamin E acetate, and vitamin E nicotinate, vitamin D's, vitamin H, pantothenic acid, and pantethine.
Various drugs such as nicotinamide, benzyl nicotinate, γ-oryzanol, allantoin, glycyrrhizic acid (salt), glycyrrhizic acid and its derivatives, hinokitiol, musidine, bisabolol, eucalyptol, thymol, inositol, saponins (saikosaponin, carrot saponin, gourd saponin, soapberry saponin, etc.) pantothenylethyl ether, ethynylestradiol, tranexamic acid, cepharanthine, and placenta extract.
Natural extracts from Rumex japonicus, Sophora flavescens, Nuphar japonica, orange, sage, thyme, yarrow, mallow, smilax, swertia, Ligusticum acutilobum, bitter orange peel, birch, horsetail, gourd, horse chestnut, creeping saxifrage, arnica, lily, mugwort, Paeonia lactiflora, aloe, gardenia, Chamaecyparis pisifera, etc. extracted by using an organic solvent, alcohol, polyhydric alcohol, water, hydroalcohol, etc.
Cation surfactants such as stearyltrimethylammonium chloride, benzalkonium chloride, and lauryl amine oxide.
Sequestering agents such as disodium edetate, trisodium edetate, sodium citrate, sodium polyphosphate, sodium metaphosphate, and gluconic acid.
Perfumes, scrubbing agents, purified water, etc.

Particularly preferable base agents for the sunscreen cosmetic of the present invention are oil components including decamethylcyclopentasiloxane, isononyl isononanoate, dimethylpolysiloxane, heptamethyloctyltrisiloxane, trimethylsiloxysilicic acid, liquid paraffin, squalane, cetyl isooctanoate, triglyceride octanoate, and di-2-ethylhexyl succinate. The present invention is used preferably in sunscreen cosmetics that use decamethylcyclopentasiloxane as the main base agent.

The sunscreen cosmetic of the present invention can be used in any product form such as ointments, emulsion, lotions, and packs. The dosage form is not limited either.

### EXAMPLES

The invention is described in specific detail through Examples. The present invention is not limited to these Examples.

### [First invention]

### <Synthesis example of the benzotriazole derivative represented by general formula (1-I)>

### "Synthesis example 1-1: Synthesis of 2-[4-(2-ethylhexyloxy)-2-hydroxyphenyl]-2H-benzotriazole"

45.4 g (0.20 moles) of 6-(2H-benzotriazole-2-yl) resorcinol, synthesized by using a conventional method, was put into a 500 ml four neck flask equipped with a thermometer and a reflux cooling apparatus, to which 50 ml of methylisobutylketone and 4.0 g of dimethylformamide were added, followed by stirring. Into this, 25.4 g (0.24 moles) of sodium carbonate and 77.2 g (0.40 moles) of 2-ethylhexyl bromide were added and the mixture was heated up to the refluxing temperature while being stirred. After stirring the mixture for 15 hours while maintaining the refluxing temperature, methylisobutylketone was recovered under normal pressure and the remaining oil was then rinsed with water to remove excess sodium carbonate and inorganic byproducts. This oil was distilled under a reduced pressure to obtain 52.1 g of a 220-225° C/0.2-0.3 mmHg fraction, which was yellow and transparent. This compound was liquid at ordinary temperatures; the yield was 76.7% and the HPLC purity was 99.0%.

### "Synthesis example 1-2: Synthesis of 2-(2-hydroxy-4-isobutoxyphenyl)-2H-benzotriazole"

Instead of 2-ethylhexyl bromide, the equal number of moles of isobutyl bromide was used in the same manner as in Synthesis example 1. Slightly yellow-gray-white powdery crystals were obtained at a yield of 72.5%. The m. p. was 120.0-120.8° C, λₘₐₓ = 345.6 nm, and ε = 21750.

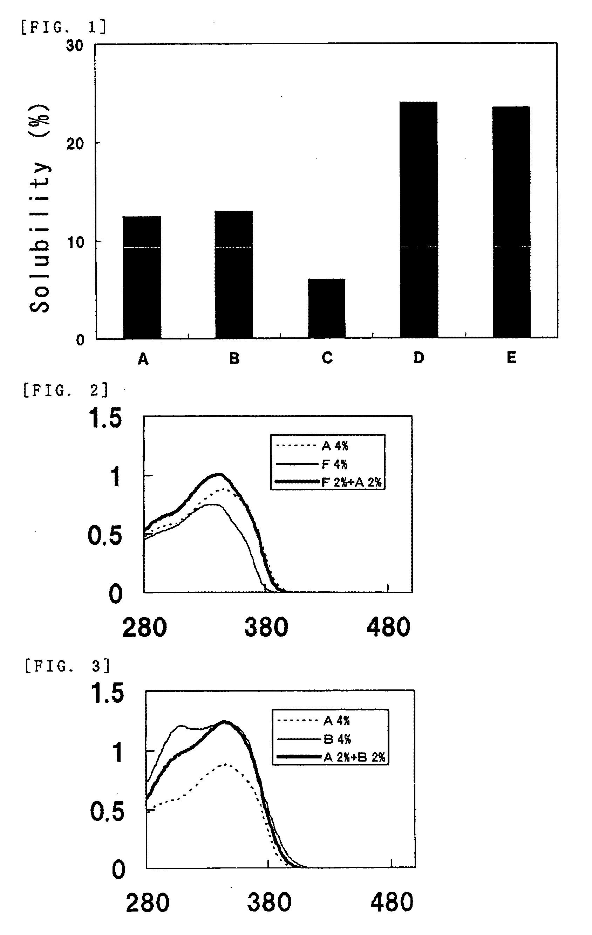

Solubility of 2,4-bis [[4-(2-ethylhexyloxy)-2-hydroxy] phenyl]-6-(4-methoxyphenyl)-1,3,5-triazine in the following oil components A-E was measured. The results are shown in FIG. 1.
A: Isodecyl benzoate
B: 2-ethylhexyl benzoate
C: Ethylhexyl 2-cyano-3,3-diphenylacrylate (octocrylene)

### <Oil component described in Patent Document 2>

D: 2-[4-(2-ethylhexyloxy)-2-hydroxyphenyl]-2H-benzotriazole

### <Oil component of Synthesis example 1-1>

E: 2-(2-hydroxy-4-isobutoxyphenyl)-2H-benzotriazole

### <Oil component of Synthesis example 1-2>

### "Method and conditions for measuring the solubility"

2,4-bis [[4-(2-ethylhexyloxy)-2-hydroxy] phenyl]-6-(4-methoxyphenyl)-1,3,5-triazine is heated and dissolved at 80°C in each of the aforementioned oil components A-E. After the temperature naturally cools down to room temperature, a small amount of 2,4-bis [[4-(2-ethylhexyloxy)-2-hydroxy] phenyl]-6-(4-methoxyphenyl)-1,3,5-triazine in powder form is added, followed by three days of stirring and cooling (stirrer) at 0°C. After this, the supernatant solution is quickly sampled before the temperature rises and 30 mg (actual measurement) of it is diluted to 100 mL of ethyl acetate. The absorbance at 342.8 nm of the obtained diluted solution is measured. The obtained absorbance is plotted against a previously prepared calibration curve to determine the solubility.

FIG. 1 indicates that the solubility is particularly good in the benzotriazole derivatives D and E of general formula (1-I).
Therefore, a sunscreen cosmetic containing 2,4-bis [[4-(2-ethylhexyloxy)-2-hydroxy] phenyl]-6-(4-methoxyphenyl)-1,3,5-triazine and a benzotriazole of general formula (1-I) is superior in terms of the solubility of 2,4-bis [[4-(2-ethylhexyloxy)-2-hydroxy] phenyl]-6-(4-methoxyphenyl)-1,3,5-triazine and as a result it is possible to provide a sunscreen cosmetic that allows said ultraviolet absorbent to exhibit sufficient UV-A ultraviolet absorption capacity, prevents precipitation of said ultraviolet absorbent, and exhibits superior product stability.
Also, the benzotriazole derivative of general formula (1-I), i.e. the solubilizing oil component, has its own superior UV-A ultraviolet absorption capacity; therefore together they can manifest high ultraviolet absorption capacity. Because of this, there is also an advantage in that the blend ratio of the ultraviolet absorbents can be smaller compared with other sunscreen cosmetics and still manifest the same degree of ultraviolet absorption capacity.

The following are formulation examples of the sunscreen cosmetic of the present invention that has the aforementioned effects.

**"Example 1-1: Sunscreen cosmetic, W/0 emulsion"**

| | wt% |
|---|---|
| 1. Dimethylpolysiloxane | 1 |
| 2. Decamethylcyclopentasiloxane | 25 |
| 3. Trimethylsiloxysilicic acid | 5 |
| 4. Polyoxyethylene-methylpolysiloxane copolymer | 1 |
| 5. Lauryl PEG-9 polydimethylsiloxyethyl dimethicone | 1 |
| 6. Isononyl isononoate | 5 |
| 7. Dipropylene glycol | 5 |
| 8. Dipotassium glycyrrhizate | 0.02 |
| 9. Glutathione | 1 |
| 10. Thiotaurine | 0.05 |
| 11. Sophora flavescens extract | 1 |
| 12. Paraben | Appropriate amount |
| 13. Phenoxyethanol | Appropriate amount |
| 14. 2-ethylhexyl paramethoxycinnamate | 7.5 |
| 15. Dimethyldistearylammonium hectorite | 0.5 |
| 16. Spherical polyalkyl acrylate powder | 5 |
| 17. Butylethylpropanediol | 0.5 |
| 18. 2,4-bis{[4-(2-ethylhexyloxy)-2-hydroxy]phenyl}-6-(4-methoxyphenyl)1, 3, 5-triazine | 2 |
| 19. 2-[4-(2-ethylhexyloxy)-2-hydroxyphenyl]-2H-benzotriazole | 8 |
| 20. Hydrophobicized zinc oxide or hydrophobicized titanium oxide | 15 |
| 21. Purified water | Balance |

### Preparation method

The water phase was gradually added to the oil phase; after the addition a stirrer was used to homogenize the emulsified particles to complete the preparation.

**"Example 1-2: Sunscreen cosmetic, O/W emulsion"**

| | wt% |
|---|---|
| 1. Polyoxyethylene hydrogenated castor oil | 1 |
| 2. Dimethicone polyol | 0.5 |
| 3. Decamethylcyclopentasiloxane | 15 |
| 4. Isostearic acid | 0.5 |
| 5. Phenyl trimethicone | 1 |
| 6. Hydrophobicized titanium oxide | 5 |
| 7. 2-ethylhexyl-paramethoxycinnamate | 3 |
| 8. 2,4-bis{[4-(2-ethylhexyloxy)-2-hydroxy]phenyl}-6-(4-methoxyphenyl)1, 3, 5-triazine | 2 |
| 9. 2-(2-hydroxy-4-isobutoxyphenyl)-2H-benzotriazole | 5 |
| 10. Silica | 1 |
| 11. Citric acid | 0.01 |
| 12. Sodium citrate | 0.09 |
| 13. Paraben | Appropriate amount |
| 14. Phenoxyethanol | Appropriate amount |
| 15. Alcohol | 5 |
| 16. Dynamite glycerin | 1 |
| 17. Succinoglucan | 0.2 |
| 18. Cellulose gum | 1 |
| 19. Ion-exchanged water | Balance |

### Preparation method

After preparing the water phase, 9-18, it was gradually added to the oil phase, 1-8, followed by stirring by means of a homomixer.

**"Example 1-3: Sunscreen cosmetic, O/W/O emulsion"**

| | wt% |
|---|---|
| 1. Polyoxyethylene hydrogenated castor oil | 0.5 |
| 2. Isostearic acid | 0.2 |
| 3. 2-ethylhexyl paramethoxycinnamate | 7.5 |
| 4. 2-[4-(2-ethylhexyloxy)-2-hydroxyphenyl]-2H-benzotriazole | 3.0 |
| 5. 2,4-bis{[4-(2-ethylhexyloxy)-2-hydroxy]phenyl}-6-(4-methoxyphenyl)1, 3, 5-triazine | 3.0 |
| 6. Decamethylcyclopentasiloxane | 35 |
| 7. Polyoxybutylene polyoxypropylene glycol | 2.0 |
| 8. Dimethyldistearylammonium hectorite | 0.5 |
| 9. Hydrophobicized titanium oxide | 12.0 |
| 10. Citric acid | 0.04 |
| 11. Sodium citrate | 0.06 |
| 12. Dipropylene glycol | 2.0 |
| 13. Methyl glucose | 1.0 |
| 14. Dynamite glycerin | 1.0 |
| 15. Sodium chloride | 0.1 |
| 16. Methylparaben | Appropriate amount |
| 17. Phenoxyethanol | Appropriate amount |
| 18. Ion-exchanged water | Balance |

### Preparation method

The oil phase consisting of 1-5 was gradually added to the water phase, 10-18, to obtain an O/W preparation. This preparation was gradually added to the oil phase consisting of 6-9, followed by stirring using a homomixer to obtain a target sample.

### [Second invention]

### <Synthesis example of the benzotriazole derivative represented by general formula (2-I)>

### "Synthesis example 2-1: Synthesis of 2-[4-(2-ethylhexyloxy)-2-hydroxyphenyl]-2H-benzotriazole"

45.4 g (0.20 moles) of 6-(2H-benzotriazole-2-yl) resorcinol, synthesized by using a conventional method, was put into a 500 ml four neck flask equipped with a thermometer and a reflux cooling apparatus, to which 50 ml of methylisobutylketone and 4.0 g of dimethylformamide were added, followed by stirring. Into this, 25.4 g (0.24 moles) of sodium carbonate and 77.2 g (0.40 moles) of 2-ethylhexyl bromide were added and the mixture was heated up to the refluxing temperature while being stirred. After stirring the mixture for 15 hours while maintaining the refluxing temperature, methylisobutylketone was recovered under normal pressure and the remaining oil was then rinsed with water to remove excess sodium carbonate and inorganic byproducts. This oil was distilled under a reduced pressure to obtain 52.1 g of a 220-225° C/0.2-0.3 mmHg fraction, which was yellow and transparent. This compound was liquid at ordinary temperatures; the yield was 76.7% and the HPLC purity was 99.0%.

### "Synthesis example 2-2: Synthesis of 2-[2-hydroxy-4-isobutoxyphenyl]-2H-benzotriazole"

Instead of 2-ethylhexyl bromide, the equal number of moles of isobutyl bromide was used in the same manner as in Synthesis example 2-1. Slightly yellow-gray-white powdery crystals were obtained at a yield of 72.5%. The m. p. was 120.0-120.8° C, λₘₐₓ = 345.6 nm, and ε = 21750.

### <Synthesis example of the alkylaryl-1,3-propanedione silicone derivative represented by general formula (2-II) >

Examples of specific preferable compounds include the following 2-1 to 2-9; of these, preparation examples of compound 2-1, compound 2-2, compound 2-5, compound 2-7, compound 2-8, and compound 2-9 are shown.
"Compound 2-1" 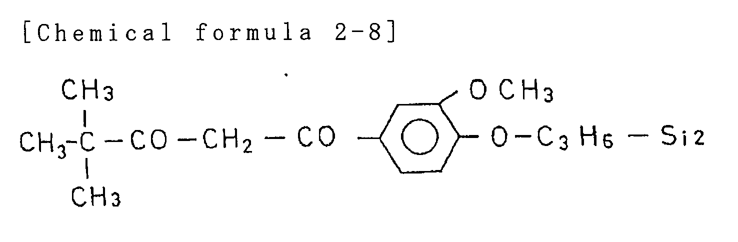
"Compound 2-2" 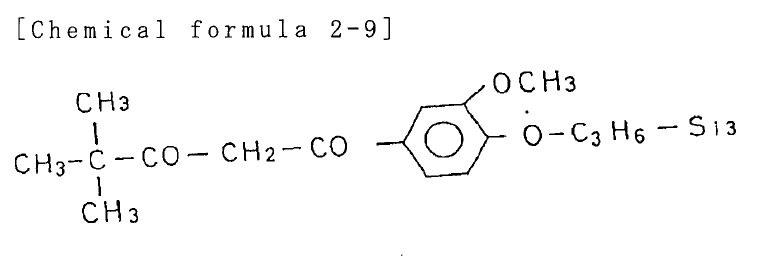
"Compound 2-3" 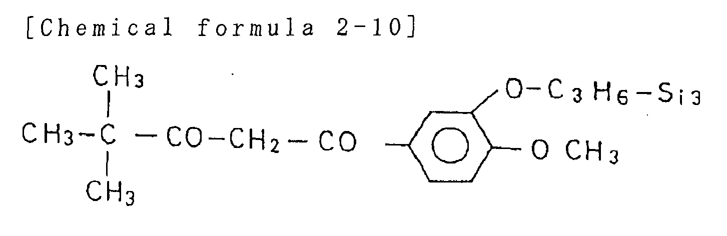
"Compound 2-4" 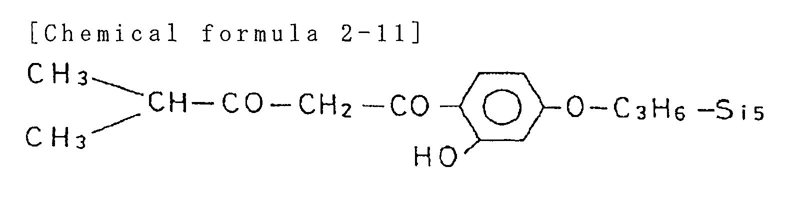
"Compound 2-5" 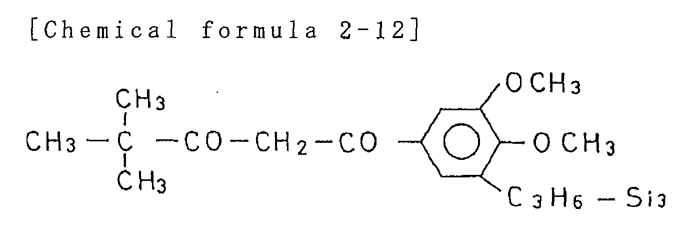
"Compound 2-6" 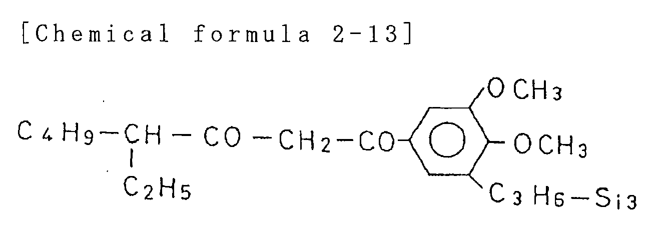
"Compound 2-7" 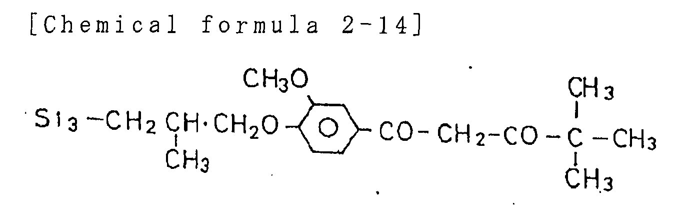
"Compound 2-8" 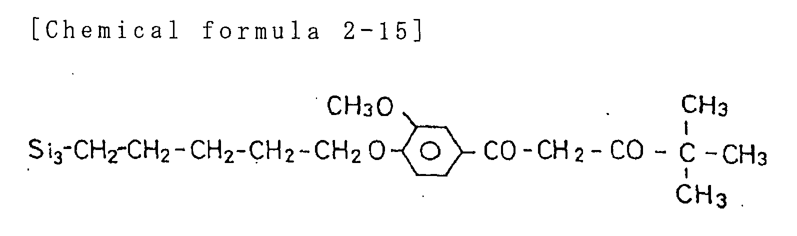
"Compound 2-9" 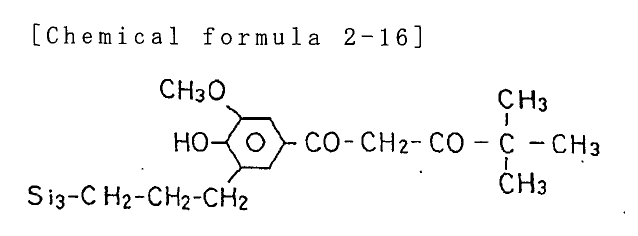
Si₂, Si₃, and Si₅ in the aforementioned compounds 2-1 to 2-9 are represented by the following chemical formulas, respectively.
"Si₂" 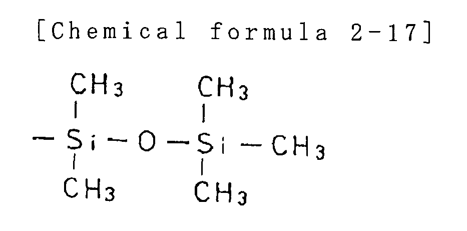
"Si₃" 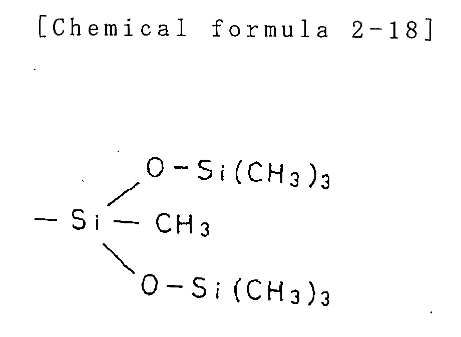
"Si₅"

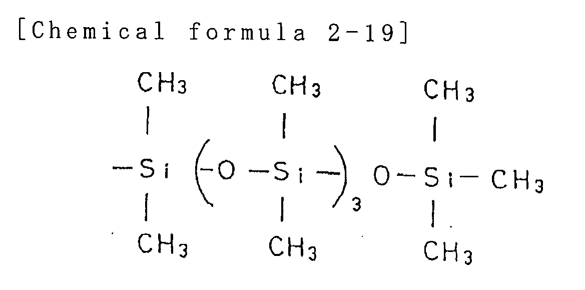

### "Preparation example of Compound 2-1"

0.5 mg of hydrogen hexachloroplatinate (IV) hexahydrate was added to a mixture of 2.5 g of 1-(3-methoxy-4-allyloxyphenyl)-3-tert-butyl-1,3-propanedione, 1.7 g of 1,1,1,3,3-pentamethyldisiloxane, and 20 ml of toluene and reacted for 6 hours at 100-105°C while being stirred. After the completion of the reaction, toluene was removed under a reduced pressure and the residual was separated and purified by means of silica gel column chromatography (eluted with 2 v/v% ethyl acetate-hexane mixture) to obtain 2.6 g of a colorless oil-like substance. The yield was 68. 4%. λₘₐₓ : 335 nm (ε = 24090), mass spectrum M⁺m/e 438

### "Preparation example of Compound 2-2"

0.03 g of a toluene solution of tetramethyldivinyldisiloxane platinum complex (platinum content 4 w/w%) was added to a solution of 13.0 g of 1-(3-methoxy-4-allyloxyphenyl)-3-tert-butyl-1,3-propanedione, 11.0 g of 1,1,1,3,5,5,5-heptamethyltrisiloxane, and 30 ml of toluene, followed by refluxing and stirring for 5 hours. After the completion of the reaction, toluene was removed under a reduced pressure and the residual was separated and purified by means of silica gel chromatography (eluted with 2 v/v% ethyl acetate-hexane mixture) to obtain 10.2 g of the target substance. The yield was 47.1%. λₘₐₓ : 334 nm (ε = 23550), mass spectrum M⁺m/e 512

### "Preparation example of Compound 2-5"

0.01 g of a toluene solution of tetramethylvinyldisiloxane platinum complex (platinum content 4 w/w%) was added to a solution of 3.4 g of 1-(3-allyl-4,5-dimethoxyphenyl)-3-(1-ethylpentyl)-1,3-propanedione, 2.4 g of 1,1,1,3,5,5,5-heptamethyltrisiloxane, and 20ml of toluene, and reacted for 6 hours while being refluxed. After the completion of the reaction, toluene was removed under a reduced pressure and the residual was put through a silica gel column. 1 v/v ethyl acetate-hexane mixture was used for elution and 4.1 g of the target substance was obtained. The yield was 73.2%.
Colorless oil λₘₐₓ = 334 nm (ε = 23500), mass spectrum M⁺m/e 568

### "Preparation example of Compound 2-7"

0.05 g of the platinum catalyst described in Example 2-2 was added to a solution consisting of 25.1 g of 1-{3-methoxy-4-(2-methyl-2-propenoxy) phenyl}-3-tert-butyl-1,3-propanedione, 20.2 g of 1,1,1,3,5,5,5-heptamethyltrisiloxane, and 50 ml of toluene, and reacted for 18 hours at 100-110°C. After the completion of the reaction, toluene was removed by means of distillation, and the residual was distilled under a reduced pressure to separate and purify the target substance. Yield 28.8 g, light yellow oil-like, boiling point 182-184°C/2 mmHg, λₘₐₓ: 334 nm (ε = 27350), mass spectrum M⁺m/e 526

### "Preparation example of Compound 2-8"

38.7 g of 1-{3-methoxy-4-(4-pentenoxy) phenyl}-3-tert-butyl-1,3-propanedione, 29.7 g of 1,1,1,3,5,5,5-heptamethyltrisiloxane, and 0.02 g of the platinum catalyst described in Example 2-2 were dissolved in 70 ml of toluene, and reacted for 4 hours at 95-100°C. After the completion of the reaction, toluene was removed by means of distillation, and the residual was distilled under a reduced pressure to obtain 39.7 g of the target compound. Light yellow oil-like, boiling point 215-225°C (bath-heated)/2 mmHg, λₘₐₓ: 334 nm (ε = 23220), mass spectrum M⁺m/e 540

### "Preparation example of Compound 2-9"

18.4 g of 1-(3-methoxy-4-hydroxy-5-allylphenyl)-3-tert-butyl-1,3-propanedione (light yellow crystals, melting point 115-116°C) obtained from the Claisen reaction of 1-(3-methoxy-4-allyloxyphenyl)-3-tert-butyl-1,3-propanedione, 15.5 g of 1, l, 1, 3, 5, 5, 5-heptamethyltrisiloxane, and 0.02 g of the platinum catalyst described in Example 2-2 were dissolved in 50 ml of toluene and reacted for 6 hours at 100-110°C. After the completion of the reaction, toluene was removed by means of distillation and the residual was separated and purified by using silica gel column chromatography (eluted with 7 v/v% ethyl acetate-hexane mixture) to obtain 21.7 g of the target substance. Light yellow solid, melting point 38-39°C, λₘₐₓ : 342 nm (ε = 24000), mass spectrum M⁺m/e 512

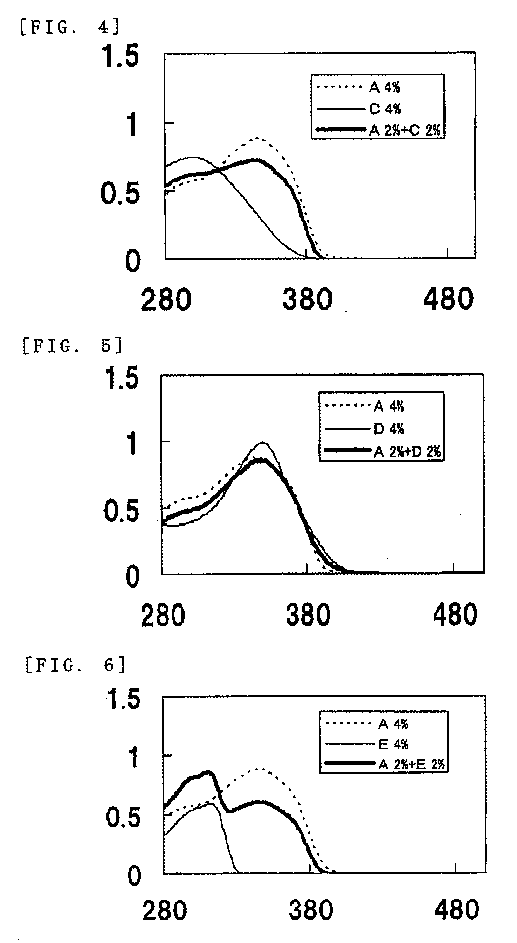

Ultraviolet absorption characteristics of the ultraviolet absorbents shown in the following Table 2-1 were investigated. The results are shown in the ultraviolet absorption spectra in FIG. 2 to FIG. 6.

### "Method and conditions for the measurement"

Each of the ultraviolet absorbents, in the amount shown in Table 2-1, was heated/dissolved in glyceryl tri-2-ethylhexanoate at 70°C. After the temperature was lowered down to room temperature, 50 ul of the dissolved sample was applied and evenly spread on a 5 x 5 cm nylon thin film. After 15 minutes, the absorbance was measured with a spectrophotometer.

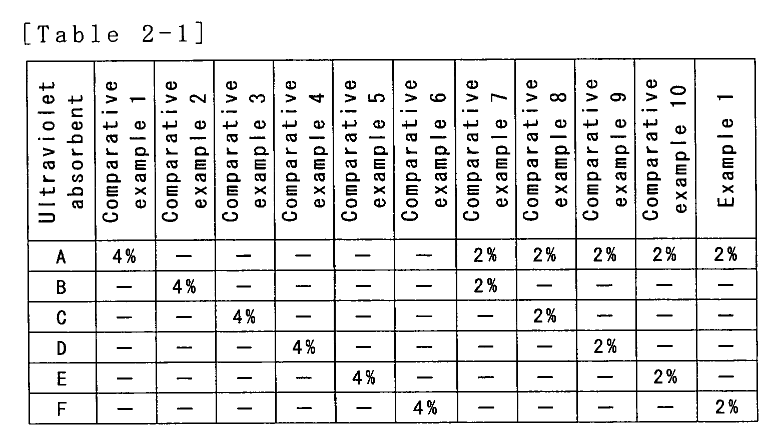

Ultraviolet absorbent A: 2-[4-(2-ethylhexyloxy)-2-hydroxyphenyl]-2H-benzotriazole {Benzotriazole derivative represented by general formula (2-I)} 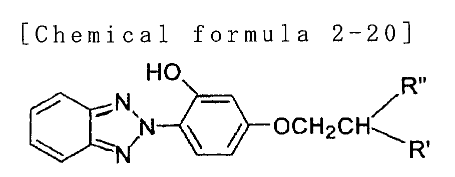 R' : Butyl group (C4 straight chain alkyl group), R": Ethyl group (C2 straight chain alkyl group)
Ultraviolet absorbent B: 2,4-bis{[4-(2-ethylhexyloxy)-2-hydroxy]phenyl}-6-(4-methoxyphenyl)-1,3,5-triazine 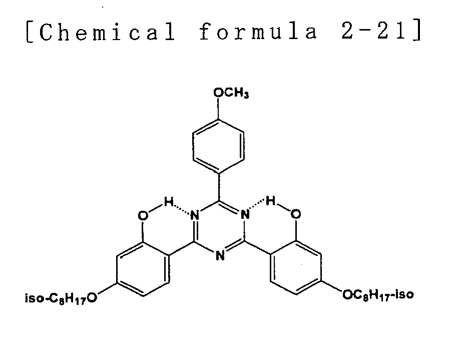
Ultraviolet absorbent C: Ethylhexyl 2-cyano-3,3-diphenylacrylate 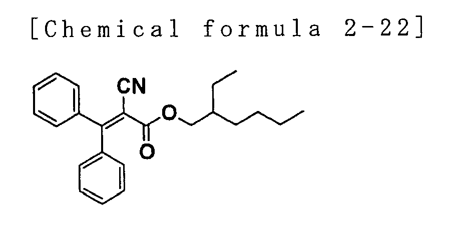
Ultraviolet absorbent D: Diethlyaminohydroxybenzoyl hexyl benzoate 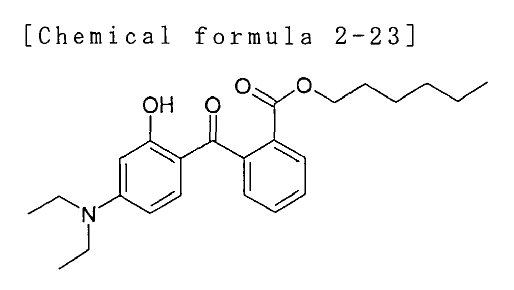
Ultraviolet absorbent E: 2,4,6-tris [p-((2'-ethylhexyl) oxycarbonyl) anilino]-1,3,5-triazine 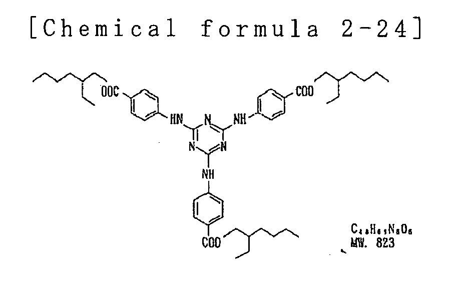
Ultraviolet absorbent F: 1-(3-methoxy-4-allyloxyphenyl)-3-tert-butyl-1,3-propanedione {Alkylaryl-1,3-propanedione silicone derivative represented by general formula (2-II)}

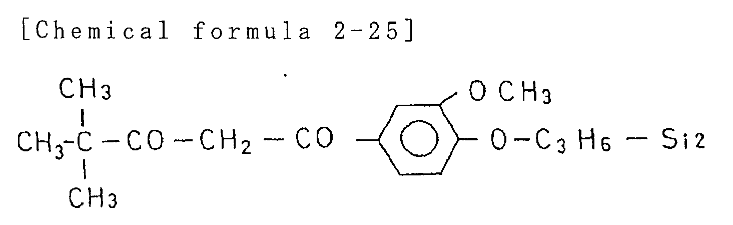

FIG. 2 shows ultraviolet absorption spectra (absorbance) comparing the absorption characteristics of a combination of the benzotriazole derivative of general formula (2-I) and the alkylaryl-1,3-propanedione silicone derivative of general formula (2-II) and the absorption characteristics of each of them independently.
FIG. 3 to FIG. 6 show ultraviolet absorption spectra (absorbance) comparing the absorption characteristics of a combination of other dibenzoylmethane derivative ultraviolet absorbents and the absorption characteristics of each of them independently.
FIGs 2 to 6 indicates that, in terms of combinations of ultraviolet absorbents composed of dibenzoylmethane derivatives, the combination of the benzotriazole derivative of general formula (2-I) and the alkylaryl-1,3-propanedione silicone derivative of general formula (2-II) exhibits synergistically pronounced ultraviolet absorption characteristics in the UV-A and UV-B regions, i.e. the 280-400 nm range, compared with respective independent absorption characteristics.

The following are formulation examples of the sunscreen cosmetic of the present invention.

**"Example 2-2: Sunscreen cosmetic, W/O emulsion"**

| | wt% |
|---|---|
| 1. Dimethylpolysiloxane | 1 |
| 2. Decamethylcyclopentasiloxane | 25 |
| 3. Trimethylsiloxysilicic acid | 5 |
| 4. Polyoxyethylene-methylpolysiloxane copolymer | 1 |
| 5. Lauryl PEG-9 polydimethylsiloxyethyl dimethicone | 1 |
| 6. Isononyl isononoate | 5 |
| 7. Dipropylene glycol | 5 |
| 8. Dipotassium glycyrrhizate | 0.02 |
| 9. Glutathione | 1 |
| 10. Thiotaurine | 0.05 |
| 11. Sophora flavescens extract | 1 |
| 12. Paraben | Appropriate amount |
| 13. Phenoxyethanol | Appropriate amount |
| 14. 2-ethylhexyl paramethoxycinnamate | 7.5 |
| 15. Dimethyldistearylammonium hectorite | 0.5 |
| 16. Spherical polyalkyl acrylate powder | 5 |
| 17. Butylethylpropanediol | 0.5 |
| 18. Benzotriazole derivative on Synthesis example 2-2 | 3 |
| 19. Alkyl-aryl 1,3-propanedione silicone derivative of formula (2-IV) | 3 |
| 20. Hydrophobicized zinc oxide or hydrophobicized titanium oxide | 15 |
| 21. Purified water | Balance |

### Preparation method

The water phase was gradually added to the oil phase; after the addition a stirrer was used to homogenize the emulsified particles to complete the preparation.

**"Example 2-3: Sunscreen cosmetic O/W emulsion"**

| | wt% |
|---|---|
| 1. Polyoxyethylene hydrogenated castor oil | 1 |
| 2. Dimethicone polyol | 0.5 |
| 3. Decamethylcyclopentasiloxane | 15 |
| 4. Isostearic acid | 0.5 |
| 5. Phenyl trimethicone | 1 |
| 6. Hydrophobicized titanium oxide | 5 |
| 7. 2-ethylhexyl-paramethoxycinnamate | 3 |
| 8. Benzotriazole derivative on Synthesis example 2-1 | 2 |
| 9. Alkyl-aryl 1,3-propanedione silicone derivative of formula (2-III) | 5 |
| 10. Silica | 1 |
| 11. Citric acid | 0.01 |
| 12. Sodium citrate | 0.09 |
| 13. Paraben | Appropriate amount |
| 14. Phenoxyethanol | Appropriate amount |
| 15. Alcohol | 5 |
| 16. Dynamite glycerin | 1 |
| 17. Succinoglucan | 0.2 |
| 18. Cellulose gum | 1 |
| 19. Ion-exchanged water | Balance |

### Preparation method

After preparing the water phase, 9-19, it was gradually added to the oil phase, 1-8, followed by stirring by means of a homomixer.

**"Example 2-4: Sunscreen cosmetic, O/W/O emulsion"**

| | wt% |
|---|---|
| 1. Polyoxyethylene hydrogenated castor oil | 0.5 |
| 2. Isostearic acid | 0.2 |
| 3. 2-ethylhexyl paramethoxycinnamate | 7.5 |
| 4. Alkyl-aryl 1,3-propanedione silicone derivative of formula (2-IV) | 3.0 |
| 5. Benzotriazole derivative on Synthesis example 2-2 | 5.0 |
| 6. Decamethylcyclopentasiloxane | 35 |
| 7. Polyoxybutylene polyoxypropylene glycol | 2.0 |
| 8. Dimethyldistearylammonium hectorite | 0.5 |
| 9. Hydrophobicized titanium oxide | 12.0 |
| 10. Citric acid | 0.04 |
| 11. Sodium citrate | 0.06 |
| 12. Dipropylene glycol | 2.0 |
| 13. Methyl glucose | 1.0 |
| 14. Dynamite glycerin | 1.0 |
| 15. Sodium chloride | 0.1 |
| 16. Methylparaben | Appropriate amount |
| 17. Phenoxyethanol | Appropriate amount |
| 18. Ion-exchanged water | Balance |

### Preparation method

The oil phase, 1-5, was gradually added to the water phase, 10-18, to obtain an O/W preparation. This preparation was gradually added to the oil phase consisting of 6-9, followed by stirring using a homomixer to obtain a target sample.

### INDUSTRIAL APPLICABILITY

### [First invention]

The sunscreen cosmetic of the present invention contains a benzotriazole derivative represented by the general formula (1-I) as the oil component to dissolve 2,4-bis [[4-(2-ethylhexyloxy)-2-hydroxy] phenyl]-6-(4-methoxyphenyl)-1,3,5-triazine, which is an excellent ultraviolet absorbent in the UV-A region, and thus this cosmetic can sufficiently dissolve said ultraviolet absorbent in the powder form. As a result, a sunscreen cosmetic that exhibits superior UV-A absorption capacity and excellent stability can be provided.

### [Second invention]

The sunscreen cosmetic of the present invention can synergistically improve the ultraviolet absorption capacity of conventional ultraviolet absorbents. As a result, a sunscreen cosmetic that exhibits high ultraviolet absorption capacity in a broad region from UV-A to UV-B can be provided.

## Claims

1. A sunscreen cosmetic comprising 2,4-bis [[4-(2-ethylhexyloxy)-2-hydroxy] phenyl]-6-(4-methoxyphenyl)-1,3,5-triazine and a benzotriazole derivative represented by the following general formula (1-I). 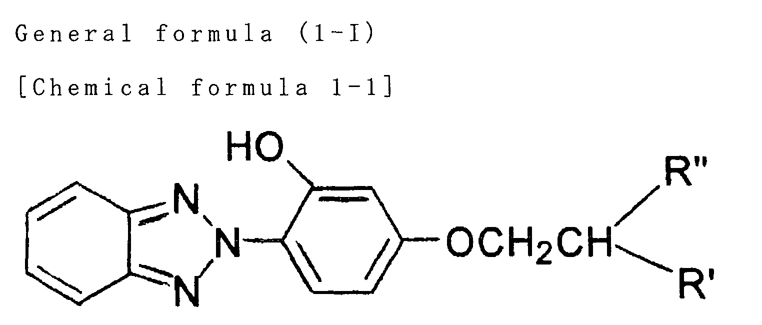 (In this formula, R' = a straight chain alkyl group of C1-C6 and R" = a straight chain alkyl group of C1-C3.)

2. The sunscreen cosmetic of claim 1 wherein said benzotriazole derivative is one, two or more chosen from a group consisting of 2-[4-(2-ethylhexyloxy)-2-hydroxyphenyl]-2H-benzotriazole and 2-(2-hydroxy-4-isobuthoxyphenyl)-2H-benzotriazole.

3. A sunscreen cosmetic comprising a benzotriazole derivative represented by the following general formula (2-I) and an alkylaryl 1,3-propanedione silicone derivative represented by the following general formula (2-II). 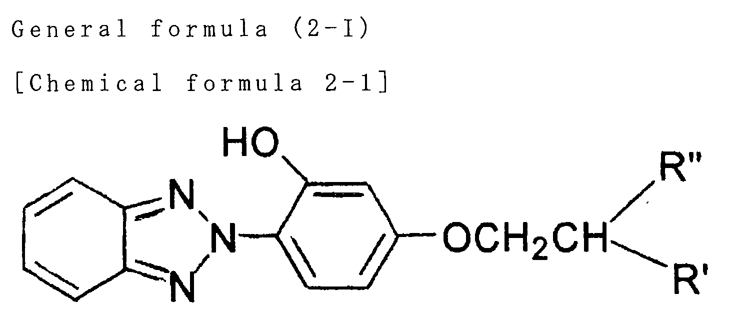 (In this formula, R' = a straight chain alkyl group of C1-C6 and R" = a straight chain alkyl group of C1-C3.) 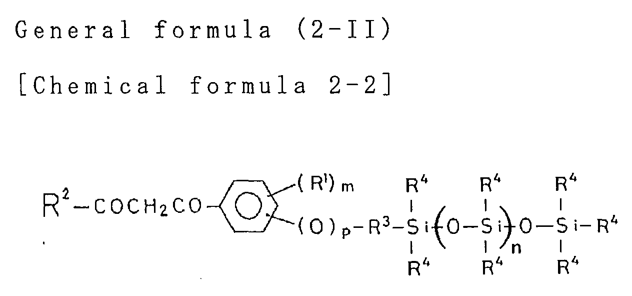 (R¹ denotes a hydroxyl group, an alkyl group having 1-8 carbons or an alkoxy group having 1-8 carbons, R² denotes an alkyl group having 1-7 carbons, R³ denotes a divalent alkylene group or oxyalkylene group having 2-10 carbons, R⁴s can be identical or different from each other and each denote an alkyl group having 1-4 carbons, phenyl group, or trimethylsiloxy group, m and n denote integers 0-3, and p denotes 0 or 1.)

4. The sunscreen cosmetic of claim 3 wherein said benzotriazole derivative is one, two or more chosen from a group consisting of 2-[4-(2-ethylhexyloxy)-2-hydroxyphenyl]-2H-benzotriazole and 2-(2-hydroxy-4-isobuthoxyphenyl)-2H-benzotriazole.

5. The sunscreen cosmetic of claim 3 or 4 wherein said alkylaryl 1,3-propanedione silicone derivative is a chemical compound represented by the following formula (2-111) or (2-IV).
